(19) Europäisches Patentamt
European Patent Office
Office européen des brevets

(11) **EP 1 649 843 B1**

(12) **EUROPEAN PATENT SPECIFICATION**

(45) Date of publication and mention
of the grant of the patent:
**12.12.2007 Bulletin 2007/50**

(51) Int Cl.:
***A61F 9/01*** *(2006.01)*

(21) Application number: **05109765.7**

(22) Date of filing: **20.10.2005**

(54) **Photoablative laser with controllable pulse release frequency**

Laser zur Photoablation mit regulierbarer Puls-Emissionsfrequenz.

Laser photoablatif avec contrôle de la fréquence d'emission des pulses.

(84) Designated Contracting States:
**DE ES FR GB IT**

(30) Priority: **22.10.2004 IT MI20042020**

(43) Date of publication of application:
**26.04.2006 Bulletin 2006/17**

(73) Proprietor: **Ligi Tecnologie Medicali S.p.A.**
**74100 Taranto (IT)**

(72) Inventor: **D'Ippolito, Giuseppe**
**74100, Taranto (IT)**

(74) Representative: **Jorio, Paolo et al**
**Studio Torta S.r.l.**
**Via Viotti, 9**
**10121 Torino (IT)**

(56) References cited:
**WO-A-99/38443          WO-A-03/101326**
**US-A- 5 984 916          US-B1- 6 302 877**

**Description**

The present invention relates to a photoablative laser.

As is known, photoablative lasers are commonly used in refractive surgery to reconstruct the cornea to correct visual defects, by removing successive layers of the cornea, varying in area, according to a predetermined ablation profile. Normally, the small-area layers are treated first, and then the larger-area layers. A photoablative laser sends pulse sequences of predetermined frequency and energy onto the cornea to locally evaporate microscopic volumes of cornea tissue. To avoid uneven ablation thickness caused by interaction between the laser spots striking the cornea and the cornea tissue evaporation fumes produced by the immediately preceding laser spots, and to prevent damage caused by overheating, the pulses are emitted to cover the layer for removal in a random as opposed to orderly sequence.

The commonly used method, however, is unsatisfactory, by only being effective as regards the large-area layers. When removing small-area layers of cornea tissue, the problems of uneven ablation thickness and overheating still remain, on account of energy accumulation still being considerable.

According to a method of ablating tissue by a scanning laser beam disclosed in WO 99/38443 A, a laser is pulsed at a faster repetition rate than allowed by a scanning mechanism, during first periods of time, and no pulses are delivered during second periods of time. In the first periods of time, scanning mirrors are stationary or moved through small regions, whereas in the second periods of time major movements take place.

Other examples of ablating arrangements and method are disclosed in WO 03/101326 A, in US-A-5 984 916 and in US-B1-6 302 877.

It is an object of the present invention to eliminate the aforementioned drawbacks.

According to the present invention, there is provided a photoablative laser as claimed in Claim 1.

A number of non-limiting embodiments of the invention will be described by way of example with reference to the accompanying drawings, in which:

Figure 1 shows a simplified block diagram of a photoablative laser in accordance with a first embodiment of the present invention;

Figure 2 shows a more detailed block diagram of part of the photoablative laser in Figure 1;

Figures 3 and 4 show diagrams of a patient's eye and a reference-axis system;

Figures 5 and 6 show simplified block diagrams of a second and third embodiment, respectively, of the present invention.

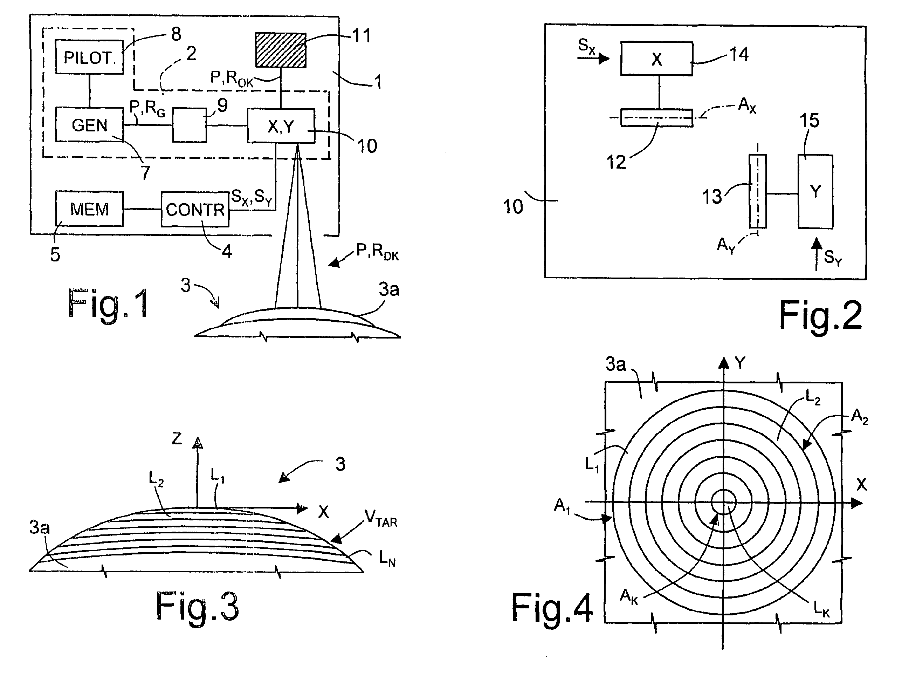

With reference to Figure 1, a photoablative laser 1 for refractive surgery comprises an apparatus 2 for emitting laser pulses P to the cornea 3a of a patient's eye 3; a control device 4 associated with apparatus 2 to control emission of laser pulses P; and a memory device 5 storing an ablation profile as defined below.

Laser pulse emission apparatus 2 comprises a laser pulse generator 7 controlled by a drive unit 8; an optical system 9; a direction device 10; and an internal target 11.

Laser pulse generator 7 is a known, e.g. excimer or solid-state type, and supplies sequences of laser pulses P of predetermined energy with a generation frequency $R_G$, preferably of over 100 Hz.

Optical system 9, which is also known, is located along the path of laser pulses P, and may, for example, comprise collimators, lens systems, filters (not shown).

Direction device 10 intercepts laser pulses P and directs them as instructed by control device 4. As shown schematically in Figure 2, direction device 10 comprises two mirrors 12, 13 located along the path of laser pulses P and orientable about respective perpendicular axes of rotation $A_X$, $A_Y$ by means of actuators 14, 15 controlled by respective direction signals $S_X$, $S_Y$ supplied by control device 4 (Figure 1).

More specifically, control device 4 is connected to memory device 5, and generates direction signals $S_X$, $S_Y$ on the basis of the ablation profile stored in the memory device.

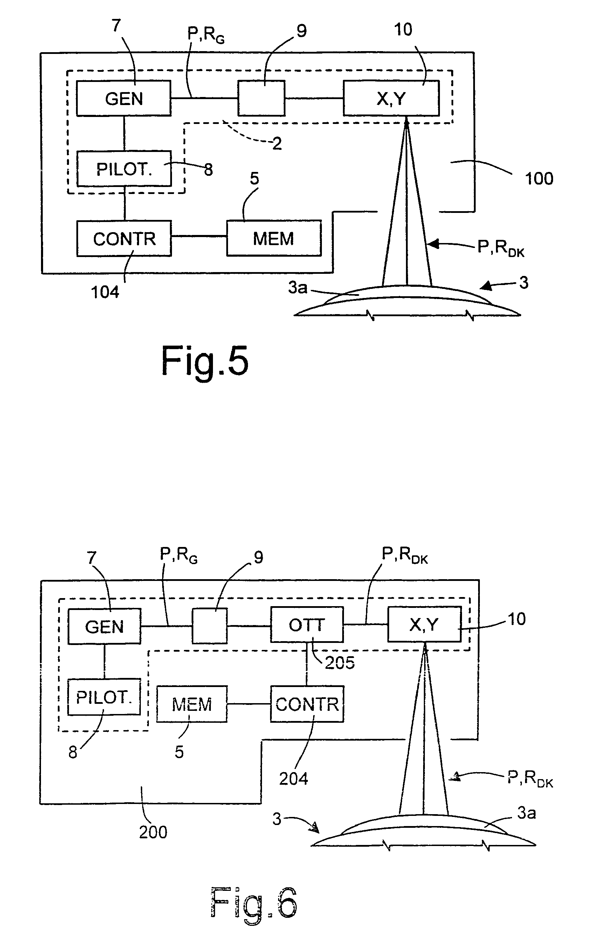

The ablation profile is defined by sets of coordinates relative to a portion of cornea tissue 3a - hereinafter referred to as the target volume $V_{TAR}$ - which must be removed to correct a refractive defect of eye 3. With reference to Figures 3 and 4, the coordinates are taken from a system of three perpendicular cartesian axes X, Y, Z, wherein the Z axis coincides with the optical axis of eye 3. In the ablation profile stored in memory device 5, target volume $V_{TAR}$ is defined in the form of a number of layers $L_1$, $L_2$, ..., $L_N$ for removal. Layers $L_1$, $L_2$, ..., $L_N$ are preferably of even thickness and respective areas $A_1$, $A_2$, ..., AN. In the Figure 3 and 4 example, the selected ablation profile calls for removing the small-area layers $L_1$, $L_2$, ..., $L_N$ first. It should be pointed out that, in the case of a particularly uneven cornea 3a, some layers may include a number of non-connected regions; and the term "layers" may also include different portions of the same physical layer of cornea 3a to be removed at different stages and therefore stored separately in memory device 5.

Under the control of control device 5, emission apparatus 2 releases laser pulses P to target volume $V_{TAR}$ with a mean release frequency $R_{DK}$. Here and hereinafter, the term "mean release frequency $R_{DK}$" refers solely to the mean frequency of the laser pulses P produced by laser pulse generator 7 and directed by direction device 10 to target volume

$V_{TAR}$ to remove a generic layer $L_K$ in the time interval between commencing ablation of generic layer $L_K$ and commencing removal of the next layer $L_1$, $L_2$, ..., $L_N$. It is also understood that the duration of the step of removing generic layer $L_K$ equals the duration of said time interval, and includes steps in which target volume $V_{TAR}$ is reached by laser pulses P, and steps in which target volume $V_{TAR}$ is not reached by laser pulses P. Mean release frequency $R_{DK}$ is therefore less than or at most equal to generation frequency $R_G$.

Control device 4 controls direction device 10 in such a way as to direct laser pulses P alternately to target volume $V_{TAR}$ and off target volume $V_{TAR}$ (preferably to internal target 11), and to control mean release frequency $R_{DK}$. In fact, the greater the number of laser pulses diverted off target volume $V_{TAR}$, the lower the mean release frequency $R_{DK}$.

More specifically, mean release frequency $R_{DK}$ is controlled as a function of areas $A_1$, $A_2$, ..., AN of layers $L_1$, $L_2$, ..., $L_N$, so that, when removing each layer $L_1$, $L_2$, ..., $L_N$, target volume $V_{TAR}$ receives a number of laser pulses P, per unit of time and per unit of area, below a predetermined threshold $N_T$. In the embodiment of the invention described here, mean release frequency $R_{DK}$ is set according to the equation:

$$R_{DK} = R_G * T_{DK} / T_{REF} = R_G * A_K / A_{REF} \qquad (1)$$

In (1), $T_{REF}$ is the time taken to ablate a reference specimen layer of area $A_{REF}$ greater than areas $A_1$, $A_2$, ..., AN of layers $L_1$, $L_2$, ..., $L_N$ at generation frequency $R_G$, to achieve a predetermined number $N_R$, below threshold $N_T$, of incident laser pulses P per unit of time and of cornea tissue area.

$$N_R \leq N_T \qquad (2)$$

$T_{DK}$ is the time taken to send the laser pulses P necessary to remove generic layer $L_K$ of area $A_K$, and is given by the equation:

$$T_{DK} = T_{REF} * AK / A_{REF} \qquad (3)$$

In the embodiment described here, the number of laser pulses P striking target volume $V_{TAR}$ per unit of time and per unit of area when removing each of layers $L_1$, $L_2$, ..., $L_N$ equals number $N_R$, is substantially constant, and is below predetermined threshold $N_T$.

Alternatively, number $N_R$ may also vary, always below threshold $N_T$, as a function of area $A_1$, $A_2$, ..., AN of layers $L_1$, $L_2$, ..., $L_N$. For example, number $N_R$ may be slightly higher to remove layers $L_1$, $L_2$, ..., $L_N$ of smaller area $A_1$, $A_2$, ..., AN.

The frequency - indicated $R_{OK}$ - with which direction device 10 diverts laser pulses P to internal target 11, on the other hand, is given by the equation:

$$R_{OK} = R_G - R_{DK} \qquad (4)$$

For each layer $L_K$, the total time $T_{OK}$ the mirrors divert the laser spots onto internal target 11 equals :

$$TOK = TREF - TDK \qquad (5)$$

When removing each layer $L_1$, $L_2$, ..., $L_N$, total time $T_{OK}$ may be an uninterrupted interval or an interval divided into a number of separate intervals.

In other words, control device 4 operates in such a way as to adapt mean release frequency $R_{DK}$ to the respective areas $A_1$, $A_2$, ..., $A_N$ of layers $L_1$, $L_2$, ..., $L_N$ as they are removed.

The photoablative laser according to the invention has the advantage of preventing uneven ablation thickness caused by interaction between the laser pulses striking the cornea and the cornea tissue evaporation fumes produced by the immediately preceding laser pulses, and of preventing overheating of the cornea tissue during treatment and any possible damage this may cause. Obviously, maximum uniformity of ablation thickness is achieved using the same number of pulses per unit of time and area for all the layers.

Figure 5 shows a second embodiment of the invention, in which any parts identical to those already described are indicated using the same reference numbers. In this case, a photoablative laser 100 comprises apparatus 2 for emitting laser pulses P; a control device 104 associated with apparatus 2 to control emission of laser pulses P; and memory device 5.

Control device 104 is connected to the drive unit 8 of laser pulse generator 7, which emits laser pulses P at a variable generation frequency $R_G$. In other words, control device 104 acts on drive unit 8 to directly control generation frequency $R_G$, and to maintain the number $N_R$ of laser pulses P sent to target volume $V_{TAR}$ per unit of time and per unit of area below predetermined threshold $N_T$. Number $N_R$ is preferably constant for all of layers $L_1$, $L_2$, ..., $L_N$. In this case, mean release frequency $R_{DK}$ equals generation frequency $R_G$. The generation frequency $R_G$ values for each layer $L_1$, $L_2$, ..., $L_N$ are set in each individual case as described above, in particular with reference to equations (1)-(5).

In the Figure 6 embodiment, the mean release frequency $R_{DK}$ of a photoablative laser 200 is controlled by a control device 204, which modifies the activation time of a shutter 205 located along the path of laser pulses P.

## Claims

1. A photoablative laser comprising:

   an ablation profile memory device (5), adapted to store sets of coordinates defining a target volume ($V_{TAR}$) for removal in the form of a number of layers ($L_1$, $L_2$, ..., $L_N$) of predetermined thickness and respective areas ($A_1$, $A_2$, ..., $A_N$);
   a laser pulse emission apparatus (2) adapted to send laser pulses (P) with a mean release frequency ($R_{DK}$) to the target volume ($V_{TAR}$) to remove said layers; and
   a control device (4; 104; 204) associated with the laser pulse emission apparatus (2) adapted to control the mean release frequency ($R_{DK}$) of the laser pulses as a function of the respective areas ($A_1$, $A_2$, ..., $A_N$) of the layers ($L_1$, $L_2$, ..., $L_N$), so that, when removing each layer, the target volume ($V_{TAR}$) receives a number ($N_R$) of laser pulses (P) per unit of time and per unit of area below a predetermined threshold ($N_T$);

   **characterized in that** the number ($N_R$) of pulses per unit of time and per unit of area is equal for all the layers ($L_1$, $L_2$, ..., $L_N$).

2. A laser as claimed in Claim 1, **characterized in that** the laser pulse emission apparatus (2) is adapted to prevent the laser pulses (P) from being sent to the target volume ($V_{TAR}$) for an uninterrupted time interval ($T_{OK}$) when removing each layer.

3. A laser as claimed in Claim 1, **characterized in that** the laser pulse emission apparatus (2) is adapted to prevent the laser pulses (P) from being sent to the target volume ($V_{TAR}$) for a number of separate time intervals when removing each layer.

4. A laser as claimed in any one of the foregoing Claims, **characterized in that** the laser pulse emission apparatus (2) comprises a laser pulse generating device adapted to (7) supply said laser pulses (P) with a generation frequency ($R_G$); and a direction device (10) adapted to direct the laser pulses.

5. A laser as claimed in Claim 4, **characterized in that** the control device (4) is connected to the direction device and adapted (10) to direct the laser pulses (P) alternately to the target volume ($V_{TAR}$) and off the target volume ($V_{TAR}$), so as to maintain below the predetermined threshold ($N_T$) the number ($N_R$) of laser pulses sent to the target volume ($V_{TAR}$) per unit of time and per unit of area when removing each layer.

6. A laser as claimed in Claim 4 or 5, **characterized in that** the direction device (10) is adapted to reduce the mean release frequency ($R_{DK}$) to less than the generation frequency ($R_G$).

7. A laser as claimed in any one of Claims 4 to 6, **characterized in that** the direction device (10) comprises two mirrors (12, 13) located along a path of said laser pulses (P) and controllable by the control device (4).

8. A laser as claimed in any one of Claims 4 to 7, **characterized in that** the control device (104) connected to the laser pulse generating device and adapted (7) to control the generation frequency ($R_G$), so that the number ($N_R$) of laser pulses sent to the target volume ($V_{TAR}$) per unit of time and per unit of area when removing each layer is maintained below the predetermined threshold ($N_T$).

**Patentansprüche**

1. Laser zur Photoablation mit einer Ablationsprofilspeichervorrichtung (5), die dazu eingerichtet ist, Sätze von Koordinaten zu speichern, die ein in der Gestalt einer Anzahl von Schichten ($L_1$, $L_2$, ..., $L_N$) von vorbestimmter Dicke und in zugehörigen Bereichen ($A_1$, $A_2$, ..., AN) zu entfernendes Zielvolumen ($V_{TAR}$) definieren, mit einer Laserpulsemissionsanordnung (2), die dazu eingerichtet ist, Laserpulse (P) mit einer mittleren Wiederholungsfrequenz ($R_{DK}$) in das Zielvolumen ($V_{TAR}$) zum Entfernen der Schichten auszusenden, und mit einer Steuervorrichtung (4; 104; 204), die mit der Laserpulsemissionsanordnung (2) zusammenwirkt und dazu eingerichtet ist, die mittlere Wiederholungsfrequenz ($R_{DK}$) der Laserpulse als eine Funktion der jeweiligen Bereiche ($A_1$, $A_2$, ..., AN) der Schichten ($L_1$, $L_2$, ..., $L_N$) zu kontrollieren, so dass beim Entfernen jeder Schicht das Zielvolumen ($V_{TAR}$) eine Anzahl ($N_R$) von Laserpulsen (P) pro Zeiteinheit und pro Flächeneinheit unterhalb einer vorbestimmten Schwelle ($N_T$) erhält, **dadurch gekennzeichnet, dass** die Anzahl ($N_R$) an Pulsen pro Zeiteinheit und pro Flächeneinheit für alle Schichten ($L_1$, $L_2$, ..., $L_N$) gleich ist.

2. Laser nach Anspruch 1, **dadurch gekennzeichnet, dass** die Laserpulsemissionsanordnung (2) dazu eingerichtet ist, das Aussenden von Laserpulsen (P) in das Zielvolumen ($V_{TAR}$) für ein ununterbrochenes Zeitintervall ($T_{OK}$) beim Entfernen jeder Schicht zu unterbinden.

3. Laser nach Anspruch 1, **dadurch gekennzeichnet, dass** die Laserpulsemissionsanordnung (2) dazu eingerichtet ist, das Aussenden von Laserpulsen (P) in das Zielvolumen ($V_{TAR}$) für eine Anzahl von einzelnen Zeitintervallen beim Entfernen jeder Schicht zu unterbinden.

4. Laser nach einem der vorangehenden Ansprüche, **dadurch gekennzeichnet, dass** die Laserpulsemissionsanordnung (2) eine Laserpulserzeugungsvorrichtung (7) aufweist, die dazu eingerichtet ist, die Laserpulse (P) mit einer Erzeugungsfrequenz ($R_G$) bereitzustellen, und über eine Ausrichtvorrichtung (10) verfügt, die dazu eingerichtet ist, die Laserpulse auszurichten.

5. Laser nach Anspruch 4, **dadurch gekennzeichnet, dass** die Steuervorrichtung (4) mit der Ausrichtvorrichtung (10) verbunden und dazu eingerichtet ist, die Laserpulse (P) abwechselnd auf das Zielvolumen ($V_{TAR}$) und neben das Zielvolumen ($V_{TAR}$) auszurichten, um die Anzahl ($N_R$) von pro Zeiteinheit und pro Flächeneinheit beim Entfernen jeder Schicht in das Zielvolumen ($V_{TAR}$) ausgesendeten Laserpulse unterhalb des vorbestimmten Schwellwertes ($N_T$) zu halten.

6. Laser nach Anspruch 4 oder 5, **dadurch gekennzeichnet, dass** die Ausrichtvorrichtung (10) dazu eingerichtet ist, die mittlere Wiederholungsfrequenz ($R_{DK}$) auf einen Wert unterhalb der Erzeugungsfrequenz ($R_G$) zu verringern.

7. Laser nach einem der Ansprüche 4 bis 6, **dadurch gekennzeichnet, dass** die Ausrichtvorrichtung (10) zwei Spiegel (12, 13) aufweist, die entlang des Weges der Laserpulse (P) angeordnet und durch die Steuervorrichtung (4) ansteuerbar sind.

8. Laser nach einem der Ansprüche 4 bis 7, **dadurch gekennzeichnet, dass** die Steuervorrichtung (104) mit der Laserpulserzeugungsvorrichtung (7) verbunden und dazu eingerichtet ist, die Erzeugungsfrequenz ($R_G$) zu kontrollieren, so dass die Anzahl ($N_R$) der pro Zeiteinheit und pro Flächeneinheit beim Entfernen jeder Schicht in das Zielvolumen ($V_{TAR}$) ausgesendeten Pulse unterhalb des vorbestimmten Schwellwertes ($N_T$) gehalten ist.

**Revendications**

1. Laser pour photoablation comprenant :

   un dispositif de mémorisation de profil d'ablation (5), conçu pour stocker des ensembles de coordonnées définissant un volume cible ($V_{TAR}$) devant être éliminé sous la forme d'un certain nombre de couches ($L_1$, $L_2$, ..., $L_N$) d'une épaisseur prédéterminée et de zones respectives ($A_1$, $A_2$, ..., $A_N$) ;
   un appareil d'émission d'impulsion laser (2) conçu pour envoyer des impulsions laser (P) ayant une fréquence de libération moyenne ($R_{DK}$) vers le volume cible ($V_{TAR}$) pour éliminer lesdites couches ; et
un dispositif de commande (4 ; 104 ; 204) associé à l'appareil d'émission d'impulsion laser (2) conçu pour commander la fréquence de libération moyenne ($R_{DK}$) des impulsions laser en fonction des zones respectives ($A_1$, $A_2$, ..., $A_N$) des couches ($L_1$, $L_2$, ..., $L_N$), de sorte que, lorsqu'on élimine chaque couche, le volume cible ($V_{TAR}$) reçoit un certain nombre ($N_R$) d'impulsions laser (P) par unité de temps et par unité de zone, inférieur à un seuil prédéterminé ($N_T$) ;

**caractérisé en ce que** le nombre ($N_R$) des impulsions par unité de temps et par unité de zone est identique pour toutes les couches ($L_1$, $L_2$, ..., $L_N$).

2. Laser selon la revendication 1, **caractérisé en ce que** l'appareil d'émission d'impulsion laser (2) est conçu pour empêcher les impulsions laser (P) d'être envoyées au volume cible ($V_{TAR}$) pendant un intervalle de temps ininterrompu ($T_{OK}$) lorsqu'on élimine chaque couche.

3. Laser selon la revendication 1, **caractérisé en ce que** l'appareil d'émission d'impulsion laser (2) est conçu pour empêcher les impulsions laser (P) d'être envoyées au volume cible ($V_{TAR}$) pendant un certain nombre d'intervalles de temps distincts lorsqu'on élimine chaque couche.

4. Laser selon l'une quelconque des revendications précédentes, **caractérisé en ce que** l'appareil d'émission d'impulsion laser (2) comprend un dispositif générateur d'impulsions laser (7) conçu pour fournir lesdites impulsions laser (P) avec une fréquence de génération ($R_G$) ; et un dispositif d'orientation (10) conçu pour diriger les impulsions laser.

5. Laser selon la revendication 4, **caractérisé en ce que** le dispositif de commande (4) est connecté au dispositif d'orientation (10) et est conçu pour diriger les impulsions laser (P) de manière alternative vers le volume cible ($V_{TAR}$) et dans une autre direction que vers le volume cible ($V_{TAR}$), de manière à maintenir sous le seuil prédéterminé ($N_T$) le nombre ($N_R$) d'impulsions laser envoyées au volume cible ($V_{TAR}$) par unité de temps et par unité de zone lorsqu'on élimine chaque couche.

6. Laser selon la revendication 4 ou 5, **caractérisé en ce que** le dispositif d'orientation (10) est conçu pour réduire la fréquence de libération moyenne ($R_{DK}$) à une valeur inférieure à la fréquence de génération ($R_G$).

7. Laser selon l'une quelconque des revendications 4 à 6, **caractérisé en ce que** le dispositif d'orientation (10) comprend deux miroirs (12, 13) situés le long d'un trajet desdites impulsions laser (P) et pouvant être contrôlés par le dispositif de commande (4).

8. Laser selon l'une quelconque des revendications 4 à 7, **caractérisé en ce que** le dispositif de commande (104) est connecté au dispositif de génération d'impulsions laser (7) et conçu pour contrôler la fréquence de génération ($R_G$), de manière à ce que le nombre ($N_R$) d'impulsions laser envoyées au volume cible ($V_{TAR}$) par unité de temps et par unité de zone lorsqu'on élimine chaque couche soit maintenu sous le seuil prédéterminé ($N_T$).

Fig.1

Fig.2

Fig.3

Fig.4

EP 1 649 843 B1

Fig.5

Fig.6

**REFERENCES CITED IN THE DESCRIPTION**

**Patent documents cited in the description**

- WO 9938443 A
- WO 03101326 A
- US 5984916 A
- US 6302877 B1